# FASCICULE DE BREVET EUROPEEN

(11) **EP 3 069 754 B1**
(45) Date de publication et mention de la délivrance du brevet: **25.10.2017**
(21) Numéro de dépôt: 16158190.5
(22) Date de dépôt: 02.03.2016
(51) Int. Cl.: A61N 1/372, A61N 1/375, A61N 1/05, A61B 17/34, A61M 25/00

(54) **ACCESSOIRE D'IMPLANTATION IN SITU POUR CAPSULE INTRACARDIAQUE AUTONOME**
IN-SITU-IMPLANTATIONSZUBEHÖR FÜR AUTONOME INTRAKARDIALE KAPSEL
IN SITU IMPLANTATION ACCESSORY FOR SELF-CONTAINED INTRACARDIAC CAPSULE

(30) Priorité: 16.03.2015 FR 1552108
(43) Date de publication de la demande: 21.09.2016
(73) Titulaire: Sorin CRM SAS, 92140 Clamart Cedex (FR)
(72) Inventeur: OLLIVIER, Jean-François, 91190 VILLIERS LE BACLE (FR); REGNIER, Willy, 91160 LONGJUMEAU (FR)
(74) Mandataire: Grünecker Patent- und Rechtsanwälte PartG mbB

(56) Documents cités:
- EP-A1- 2 394 695
- EP-A1- 2 818 201
- WO-A1-2012/082755
- US-A1- 2009 204 170

## Description

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif.

De façon générale, l'invention concerne l'implantation *in situ* de tels dispositifs lorsque ceux-ci sont pourvus à leur extrémité distale d'un organe d'ancrage du dispositif, apte à pénétrer dans les tissus d'une paroi corporelle au site d'implantation choisi.

Un exemple typique d'un tel organe d'ancrage est une vis hélicoïdale saillante prolongeant axialement le corps du dispositif médical et destinée à pénétrer dans le tissu cardiaque par vissage au site d'implantation. Ce mode d'ancrage n'est toutefois pas limitatif de l'invention, qui s'applique également à d'autres types d'organes d'ancrage, par exemple mettant en oeuvre des aiguilles, des crochets, des barbes, etc. pénétrant dans les tissus pour y assujettir de façon permanente le dispositif médical.

Selon un premier aspect, l'invention concerne plus particulièrement ceux de ces dispositifs qui se présentent sous forme d'une capsule autonome implantée dans une cavité cardiaque (ventricule, oreillette ou même cavité cardiaque gauche artérielle), ci-après désignée "capsule autonome" ou "capsule *leadless*" (le caractère autonome de la capsule n'étant toutefois pas intrinsèquement une caractéristique nécessaire de l'invention). Ces capsules autonomes sont dépourvues de toute liaison physique à un dispositif principal implanté (tel qu'un boitier de générateur d'impulsions de stimulation) ou non implanté (périphérique externe tel que programmateur ou dispositif de *monitoring* pour le suivi à distance du patient), et sont dénommées pour cette raison "capsules *leadless*", pour les distinguer des électrodes ou des capteurs disposés à l'extrémité distale d'une sonde (*lead*) conventionnelle, qui est parcourue sur toute sa longueur par un ou plusieurs conducteurs reliant par voie galvanique l'électrode ou le capteur à un générateur connecté à une extrémité opposée, proximale, de la sonde.

On verra par ailleurs que, selon un second aspect, l'invention peut être généralisée à la "délivrance", c'est-à-dire à la mise en place au site d'implantation choisi d'autres types de dispositifs médicaux, par exemple des sondes de stimulation se présentant sous forme d'un corps tubulaire portant à son extrémité distale des moyens d'ancrage à une paroi cardiaque ainsi qu'une partie active pourvue d'électrodes de détection/stimulation, et à son extrémité proximale des moyens de liaison mécaniques et électriques à un boitier de générateur, implanté à distance du site d'application des impulsions. L'invention peut s'appliquer à d'autres types encore de dispositifs implantables, par exemple à des capsules destinées à diffuser *in situ* un agent pharmacologique actif.

Dans le cas où les capsules *leadless* sont des capsules endocavitaires (c'est-à-dire des capsules à fixer à la paroi intérieure d'une cavité ventriculaire ou auriculaire, par opposition aux capsules épicardiques, fixées à la paroi extérieure du coeur), les contraintes d'implantation sont accrues du fait de la voie d'approche, qui implique de passer par le réseau veineux périphérique puis de diriger sous amplificateur de brillance la capsule vers le site d'implantation choisi, ceci de façon à la fois précise et parfaitement sécurisée. Ce n'est qu'une fois le site atteint et la capsule fermement ancrée dans la paroi cardiaque que l'opérateur pourra procéder au "largage" de cette capsule, c'est-à-dire à sa désolidarisation d'avec l'accessoire d'implantation.

Le EP 2 818 201 A1 (Sorin CRM SAS) décrit un ensemble de capsule intracardiaque avec son accessoire d'implantation *in situ.* La capsule autonome comprend un corps tubulaire cylindrique pourvu à son extrémité distale d'un organe d'ancrage apte à pénétrer dans un tissu d'une paroi d'une cavité du coeur. L'accessoire d'implantation comprend un cathéter téléorientable avec une lumière interne, prolongé à son extrémité distale par un embout tubulaire de protection définissant un volume intérieur apte à loger la capsule, des moyens découplables étant prévus pour supporter et guider la capsule jusqu'à un site d'implantation. L'accessoire d'implantation comprend en outre un sous-cathéter logé de façon mobile dans la lumière du cathéter téléorientable. Le sous-cathéter et la capsule peuvent être déployés téléscopiquement par rapport au cathéter entre une position rétractée où la capsule et son organe d'ancrage sont complètement logés à l'intérieur de l'embout tubulaire de protection, et une position déployée où la capsule est sortie de l'embout tubulaire de protection et est portée par l'extrémité distale du sous-cathéter. Enfin l'extrémité distale du sous-cathéter et la région proximale de la capsule sont pourvues de moyens de solidarisation en translation et en rotation mutuelles, ces moyens de solidarisation étant découplables pour larguer la capsule une fois en place.

Ce document décrit également bien les contraintes liées à ce type de pose, et les avantages apportés par un tel ensemble. On s'y reportera en tant que de besoin.

Le EP 2 394 695 A1 décrit un accessoire d'implantation similaire, mais ne mettant pas en oeuvre de sous-cathéter télescopique. La capsule y est portée seulement par l'embout, et couplée à ce dernier par un système à vis hélicoïdale. La pose se fait par accostage contre la paroi de l'ensemble constitué par l'embout avec la capsule à l'intérieur. Après ancrage de la capsule, un mouvement de rotation imprimé à l'accessoire permet de simultanément reculer l'embout et le découpler de la capsule. Par précaution, un fil d'Ariane relie l'embout à la capsule au cas où il serait nécessaire de réintervenir pour explanter la capsule, par exemple si le site initialement choisi ne donnait pas satisfaction après un test électrique, et un autre site devait être recherché. Le fil d'Ariane permet alors de guider l'embout jusqu'à ce qu'il atteigne la capsule, sur laquelle il pourra alors être couplé à nouveau pour permettre son explantation.

Bien que ce type de dispositif donne généralement satisfaction, sa manipulation endoveineuse et endocavitaire peut générer des risques sérieux du fait de sa taille, de son facteur de forme, de la puissance de transfert d'effort nécessaire, etc. : altération des tissus veineux et/ou cardiaques, altération de la valve tricuspide voire perforation cardiaque. En effet, l'épaisseur de la paroi cardiaque à proximité immédiate de la cible classique (l'apex) peut être de l'ordre de 1 à 2 mm seulement, et selon les méthodes de pose le médecin peut être amené à toucher directement cette fine paroi avec l'embout de protection lui-même, manoeuvré à distance (abord fémoral) via un puissant cathéter téléorientable, avec la transmission d'un couple et/ou d'une poussée axiale importants.

De plus, l'utilisation de ce type de matériel est relativement peu développée, les praticiens étant plutôt habitués à manipuler des sondes de stimulation ou de défibrillation beaucoup plus souples.

La présente invention vise à proposer un ensemble du type précité, dans lequel les contraintes susceptibles d'être appliquées à l'environnement de la voie d'accès empruntée pour la pose d'une telle capsule soient significativement diminuées, diminuant ainsi également le risque de lésion ou d'accident lors de cette pose, tout en protégeant également le dispositif avec son mécanisme téléorientable.

Un autre objet de l'invention est d'atteindre cet objectif sans changements opératoires notables pour le praticien.

Un objet secondaire de l'invention est de permettre au praticien d'observer visuellement des difficultés de progression de l'ensemble constitué de l'embout et de la capsule qu'il protège, pour entreprendre si nécessaire les actions correctrices.

L'invention propose ainsi un accessoire d'implantation *in situ* d'une capsule intracardiaque autonome comprenant un corps tubulaire cylindrique pourvu à son extrémité distale d'un organe d'ancrage apte à pénétrer dans un tissu d'une paroi d'une cavité du coeur.

Cet accessoire d'implantation comprend, de manière en elle-même connue notamment d'après le EP 2 818 201 A1 précité : un cathéter téléorientable avec une lumière interne, prolongé à son extrémité distale par un embout comprenant une embase à laquelle se raccorde le cathéter et une partie cylindrique définissant un volume intérieur apte à loger la capsule ; et un sous-cathéter logé à l'intérieur de la lumière du cathéter téléorientable avec un degré de liberté en translation relative et un degré de liberté en rotation relative par rapport au cathéter téléorientable.

Le sous-cathéter et la capsule sont déployables téléscopiquement par rapport au cathéter entre i) une position rétractée où la capsule et son organe d'ancrage sont logés à l'intérieur de l'embout, et ii) une position déployée où la capsule est sortie de l'embout et est portée par l'extrémité distale du sous-cathéter, et l'extrémité distale du sous-cathéter et la région proximale de la capsule sont pourvues de moyens découplables de solidarisation en translation et en flexion mutuelles.

De façon caractéristique de l'invention, l'embout présente, entre son embase et sa partie cylindrique, une partie souple formant une articulation assurant entre l'embase et la partie cylindrique une déformabilité élastique en flexion et en compression.

Selon diverses caractéristiques subsidiaires avantageuses :
- ladite partie souple présente la forme d'un manchon en matériau élastiquement déformable, ce manchon pouvant notamment être traversé par une série d'orifices, et/ou comprendre une zone en forme de soufflet ;
- ladite partie souple présente la forme d'une cage, pouvant notamment comprendre deux bagues espacées de fixation à l'embase et à la partie cylindrique, et un ensemble de barres élastiquement déformables reliant les deux bagues l'une à l'autre dans une direction généralement axiale ;
- les contours extérieurs de la partie souple s'étendent généralement dans le prolongement des contours de la partie cylindrique dans la direction axiale de l'embout ;
- le sous-cathéter présente, au droit de la partie souple de l'embout, une région à déformation privilégiée apte à faciliter la déformation élastique de ladite partie souple tout en préservant les efforts nécessaires à la manipulation de la capsule lors de rotations ou de translations, cette région à déformation privilégiée pouvant notamment comprendre un élément tubulaire formé par l'enroulement d'une spire, aux extrémités duquel s'arriment la partie principale du sous-cathéter et une partie des moyens découplables de solidarisation avec la capsule ;
- l'embout de protection comprend de part et d'autre de la partie souple des marqueurs radio-opaques ;
- la partie souple est au moins partiellement radio-opaque.

On va maintenant décrire un exemple de mise en oeuvre de la présente invention, en référence aux dessins annexés où les mêmes références désignent d'une figure à l'autre des éléments identiques ou fonctionnellement semblables.
La Figure 1 montre en perspective la configuration générale des différents éléments d'un ensemble capsule/cathéter/embout de protection selon l'art antérieur.
La Figure 2 est une vue en perspective d'un embout selon une première forme de réalisation de l'invention.
La Figure 3 est une vue en perspective et en demi-coupe de l'embout de la Figure 2.
La Figure 4 est une vue en perspective et en demi-coupe de l'embout relié à un cathéter, recevant une capsule reliée à un sous-cathéter.
La Figure 5 est une vue en perspective d'un embout (et de l'extrémité du cathéter associé) selon une deuxième forme de réalisation de l'invention.
La Figure 6 est une vue en perspective d'un élément d'articulation de l'embout de la Figure 5.
La Figure 7 est une vue en perspective d'un embout (et de l'extrémité du cathéter associé) selon une troisième forme de réalisation de l'invention.
La Figure 8 est une vue en perspective d'un élément d'articulation de l'embout de la Figure 7.

On va maintenant décrire, à titre d'exemple, divers modes de réalisation de l'invention.

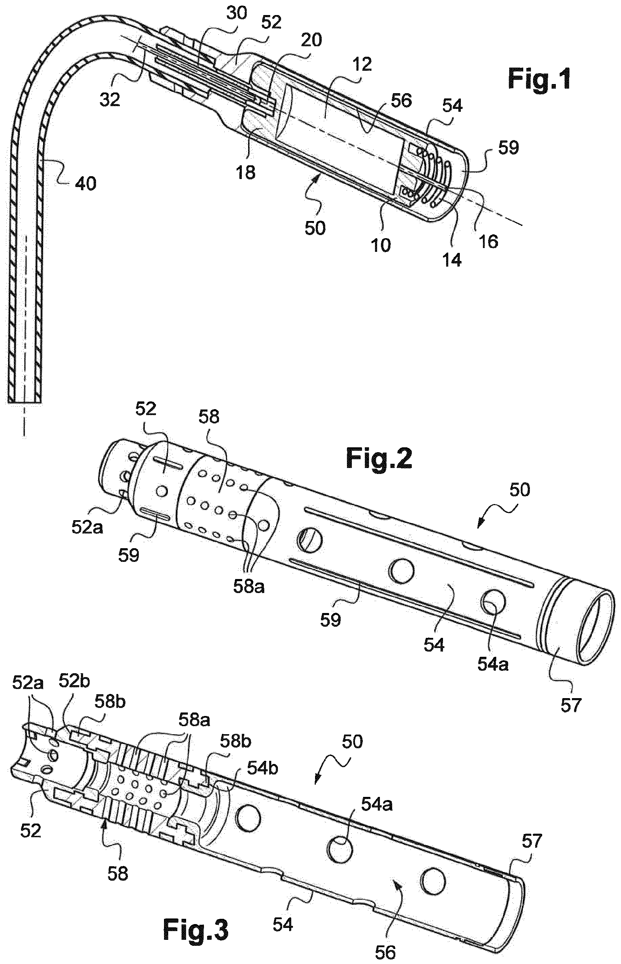

La Figure 1, qui illustre l'architecture générale du dispositif, montre un accessoire d'implantation de type connu, portant une capsule autonome de type *leadless* référencée 10.

Une telle capsule *leadless* comprend de façon en elle-même connue un corps tubulaire 12 pourvu à l'une de ses extrémités d'une vis d'ancrage 14 hélicoïdale saillante prolongeant axialement le corps tubulaire et solidaire de celui-ci en rotation. La vis d'ancrage comprend dans sa partie distale une longueur de l'ordre de 1,5 à 2 mm de spires non jointives, destinées à pénétrer dans le tissu cardiaque de façon à y assujettir la capsule. La vis 14 peut être une vis électriquement active, c'est-à-dire jouant, au moins à son extrémité distale, le rôle d'électrode de détection/stimulation, ou bien une vis passive ne servant qu'à l'ancrage du corps tubulaire 12 dans la paroi de la cavité cardiaque. Dans ce dernier cas, la capsule peut être munie d'une aiguille axiale conductrice 16 jouant le rôle d'électrode de détection/stimulation en contact avec les tissus du myocarde. En variante, il est également possible de prévoir une électrode de surface.

Le corps tubulaire 12 inclut divers moyens et circuits d'alimentation, de traitement du signal et de communication sans fil pour permettre l'échange de signaux avec un dispositif maitre distant, implanté ou non.

Ces aspects sont en eux-mêmes connus, et comme ils ne font pas partie de l'invention ils ne seront pas décrits.

À son extrémité proximale 18, le corps tubulaire 12 de la capsule 10 comprend une tige d'arrimage axial 20 comme décrit en détail dans le EP 2 818 201 A1 précité.

La capsule *leadless* 10 est destinée à être mise en place par voie basse, via la veine cave, depuis une ponction fémorale, comme décrit dans le même document.

Un cathéter téléorientable 40 est muni à son extrémité distale d'un embout tubulaire de protection 50 comportant une embase 52 à laquelle est fixée l'extrémité du cathéter 40 et une partie cylindrique 54 définissant un logement central 56 pour la capsule 10 dans une configuration dite "position rétractée" comme illustré sur la Figure 1. La principale fonction de l'embout 50 est de protéger la capsule, et notamment la vis d'ancrage 14, pendant la montée intraveineuse au passage des virages, angulations, valves, etc. Réciproquement, l'embout protège les tissus des risques de stripping potentiellement occasionnés par le mouvement de translation de la vis.

Le diamètre extérieur du cathéter téléorientable 40 est typiquement compris entre 10 et 15 French (6,6 à 10 mm), pour un diamètre intérieur de lumière compris entre 8 et 12 French (2,66 à 4 mm). Quant à l'embout tubulaire 50, il doit pouvoir loger la capsule, donc présenter un diamètre intérieur de l'ordre de 21 French (7 mm) pour une capsule du commerce actuellement fabriquée par la Demanderesse.

Le cathéter 40 est réalisé, de manière en elle-même connue, avec une structure armée, telle qu'un treillis métallique ou un bobinage noyé dans l'épaisseur de la paroi du cathéter, de manière à procurer une capacité de transmission du couple exercée sur la poignée de manoeuvre proximale jusqu'à l'extrémité distale.

L'accessoire d'implantation de l'invention comprend en outre, de façon caractéristique, un sous-cathéter 30, introduit dans la lumière centrale du cathéter téléorientable 40, et mobile en rotation et en translation par rapport à ce dernier. La fonction de ce sous-cathéter 30 est d'assurer le déploiement de la capsule hors de l'embout de protection et d'avancer cette capsule jusqu'au site d'implantation par un mouvement de translation sur une longueur suffisante, typiquement de 2 à 6 cm en fonction de l'anatomie du patient.

Le sous-cathéter 30 a également pour fonction d'assurer la transmission d'un couple de rotation depuis l'extrémité proximale (au niveau de la poignée de manoeuvre) jusqu'à son extrémité distale, et il est muni à cet effet d'une structure armée.

Il est possible d'utiliser pour la partie principale du sous-cathéter 30 un cathéter-guide conventionnel de 4 à 6 French (1,33 à 2 mm), qui est un dispositif existant, simple et économique, répondant aux contraintes courantes de transmission du couple, de faible coefficient de frottement à l'intérieur et à l'extérieur, de flexibilité, etc., et qui dispose d'une connectique "Luer-Lock" proximale permettant le montage rapide d'un adaptateur multifonction tel qu'une valve hémostatique rotationnelle ou autre adaptateur compatible avec ce standard de connexion étanche. Subsidiairement, le sous-cathéter 30 permet d'injecter un produit de contraste jusqu'à l'arrière de la capsule 10 de manière à suivre avec précision l'opération sous amplificateur de brillance.

Le moyen de couplage du sous-cathéter 30 à la capsule 10 au niveau de sa tige d'arrimage 20 est du type décrit dans le EP 2 818 201 A1 précité et ne sera pas décrit plus en détail. Optionnellement, il est prévu un fil de retenue 32 formant "fil d'Ariane", de manière également décrite dans le même document.

Grâce au moyen de couplage, le largage de la capsule peut ainsi s'effectuer comme expliqué dans le document précité, par un mouvement combiné de vissage puis de traction, en deux étapes :
- vissage de la capsule dans la paroi cardiaque, par une rotation dans le sens horaire du sous-cathéter 30, sous une légère poussée, puis
- largage de la capsule, par une poursuite de la rotation horaire du sous-cathéter 30, sous une légère traction afin de permettre le retrait du sous-cathéter.

De façon caractéristique de la présente invention, il est prévu dans une région proximale de l'embout de protection (côté proximal) une partie à flexion élastique privilégiée formant une articulation de faible rigidité.

En référence aux Figures 2 à 4, dans une première forme de réalisation cette partie, désignée par la référence 58, est constituée par un manchon en silicone souple s'étendant entre l'embase 52 et le cylindre de protection rigide 54, généralement dans l'alignement dudit cylindre.

Avantageusement, le manchon 58 est percé d'un ou de plusieurs orifices 58a permettant de diminuer sa rigidité radiale et/ou axiale. La forme et la disposition des orifices peuvent varier pour assurer la fonction attendue, par exemple afin de réduire la rigidité axiale du manchon et limiter la pression de contact frontale lorsque l'ensemble en progression rencontre une paroi. Cette forme particulière de réalisation présente également l'avantage de favoriser la circulation de sang à travers le dispositif et d'éviter la formation de caillots.

On observe à cet égard que les parties 52 et 54 de l'embout possèdent également des orifices, respectivement 52a, 54a, destinés à faciliter la circulation sanguine lors de la pose.

De façon également avantageuse, la partie proximale 52 et la partie distale 54 de l'embout sont dotées de marqueurs radio-opaques longitudinaux 59 permettant non seulement d'identifier le positionnement de l'embout en imagerie sous rayons X, mais également de constater la mise en jeu de l'articulation souple 58 (perte de l'alignement entre cathéter 40 et embout 50, ou rapprochement mutuel de ces deux zones), invitant le praticien à relâcher la contrainte si nécessaire.

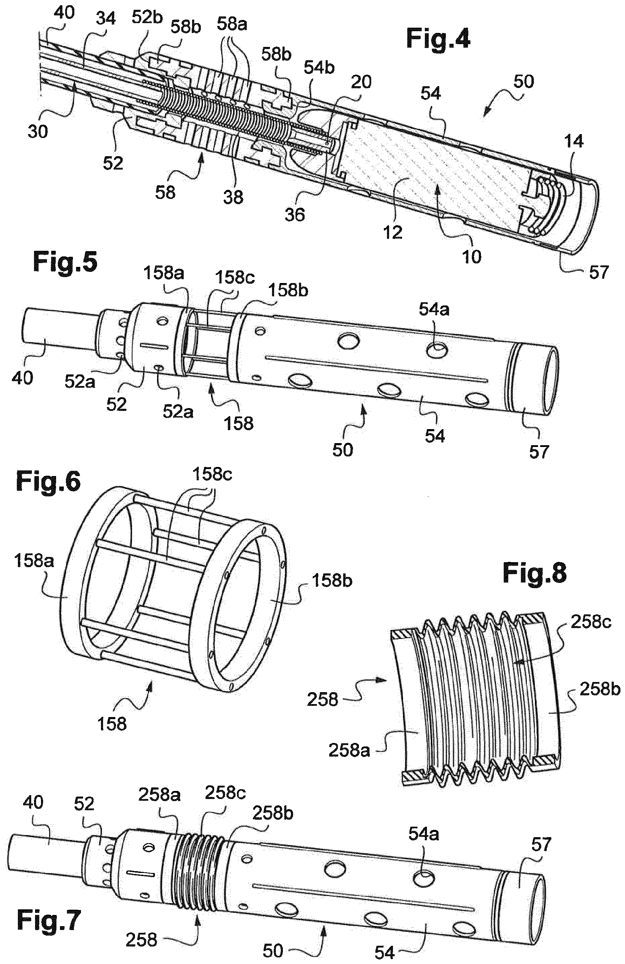

Les extrémités axiales du manchon 58 sont fixées aux éléments rigides formant respectivement l'embase 52 et le cylindre de protection 54 par exemple par collage. Comme le montrent les Figures 3 et 4, cette fixation peut être renforcée par un ancrage entre des parties mâles 58b du manchon, s'étendant localement ou sur toute l'étendue de ses bords côté embase 52 et côté cylindre 54, engagées dans des cavités homologues, respectivement 52b, 54b, de l'embase 52 et du cylindre 54, avec coopération de formes.

D'autres moyens de fixation peuvent bien entendu être mis en oeuvre.

Par ailleurs, selon une forme de réalisation possible, le matériau formant le manchon 58 est chargé de particules radio-opaques de manière à pouvoir visualiser son comportement lors de l'intervention.

En variante, le manchon 58 peut être réalisé en un autre matériau souple biocompatible, tel qu'un polyuréthanne souple.

Il est également possible, de façon connue en soi, de doter la région d'extrémité distale du cylindre 54 d'un manchon souple 57, réalisé dans le même matériau que le manchon 58 ou en un matériau différent (technologie dite *soft tip*).

Pour ne pas entraver les mouvements de flexion et de compression du manchon 58, il est important que le sous-cathéter 30 possède, au droit du manchon 58, des propriétés de déformabilité élastique au moins en flexion. En effet, sans de telles propriétés, une grande partie de l'assouplissement apporté par le manchon 58 serait perdue du seul fait de la rigidité du sous-cathéter. Cette fonction sur le cathéter peut être obtenue, dans une forme de réalisation avantageuse et comme le montre bien la Figure 4, en prévoyant dans la structure du sous-cathéter, entre sa partie principale 34 et la partie 36 d'arrimage avec la tige d'arrimage 20 de la capsule 10, une partie de liaison 38 formée par l'enroulement d'une spire jointive ou non jointive d'un fil d'un matériau (alliage métallique de préférence) de propriétés d'élasticité bien choisies.

Comme cela est visible sur la Figure 4, la partie principale 34 et la partie d'arrimage 36 du sous-cathéter s'insèrent dans la partie de liaison 38 et peuvent par exemple y être collées.

On va maintenant décrire des variantes de réalisation du manchon élastiquement déformable 58.

En référence tout d'abord aux Figures 5 et 6, le manchon, désigné par la référence 158, se présente sous la forme d'une cage, avec deux bagues circulaires 158a, 158b reliées entre elles par des barres périphériques 158c orientées axialement. Les bagues peuvent être réalisées en un matériau rigide, par exemple le même que celui des éléments rigides 52, 54 de l'embout, tandis que les barres 158c peuvent être réalisées par exemple à partir d'un microcâble d'un diamètre de l'ordre de 0,2 à 0,5 mm en un alliage métallique biocompatible ayant des propriétés d'élasticité bien choisies, tel qu'un alliage nitinol ou MP35 NLT. En variante, avec des barres de section transversale plus importante, on peut choisir de les réaliser en un matériau souple tel que silicone souple ou polyuréthanne souple.

Un tel manchon offre une souplesse en flexion et en compression, et également en rotation. Par ailleurs, les larges ouvertures existant entre les barres favorisent encore mieux que dans l'exemple précédent la circulation du sang à travers le dispositif, limitant plus efficacement encore les phénomènes de coagulation.

Une autre forme de réalisation du manchon est illustrée sur les Figures 7 et 8. Le manchon, désigné par la référence 258, présente ici la forme générale d'un soufflet 258. Plus précisément, le manchon comprend une zone principale 258c agencée sous forme d'un soufflet, réalisée d'un seul tenant avec des parties terminales en forme de bagues, plus épaisses radialement, respectivement 258a, 258b, pour la fixation du manchon respectivement à l'embase 52 et à la partie cylindrique de protection 54 de l'embout 50.

D'autres formes de réalisation du manchon sont bien entendu possibles.

Par exemple, il peut être formé d'un ressort à spires non jointives en alliage métallique, préférentiellement recouvert d'une mince couche de silicone, en particulier pour éviter tout coincement avec des tissus cardiaques ou valvulaires.

Dans tous les cas, il est avantageux que les contours extérieurs de la partie souple s'étendent généralement dans le prolongement des contours de la partie cylindrique dans la direction axiale de l'embout, afin de ne pas entraver la progression de l'embout abritant la capsule lors de sa progression le long de la voie d'accès.

La présente invention offre de nombreux avantages, parmi lesquels les suivants :
- elle confère à l'ensemble une sécurité à la pose qui est "transparente" pour le praticien, en ne nécessitant aucune action spécifique de sa part ;
- des éléments ou constituants radio-opaques sur l'embase 52 et la partie cylindrique 54, et le cas échéant sur le manchon 58, permettent une visualisation radioscopique de l'entrée en fonction de la protection (alerte visuelle pour le praticien) ;
- la configuration de l'embout selon l'invention n'affecte en rien le contrôle de la manoeuvre par le praticien (par exemple au passage de la tricuspide) ;
- cette configuration ne compromet pas le bon déroulement de la sortie progressive de la capsule (pas de blocage longitudinal ou radial de la capsule) ni de sa fixation (la déformabilité élastique locale de l'embout n'est pas susceptible d'interférer avec la pointe de la vis 14 d'ancrage de la capsule) ;
- l'ensemble reste stérilisable, présente sensiblement le même encombrement que dans l'art antérieur, et ne génère pas de surcoût significatif pour sa production ;
- des matériaux biocompatibles sont facilement disponibles pour réaliser l'articulation élastiquement déformable 58, 158, 258 de l'embout.

## Revendications

1. Un accessoire d'implantation *in situ* d'une capsule intracardiaque autonome (10) comprenant un corps tubulaire cylindrique (12) pourvu à son extrémité distale d'un organe d'ancrage (14) apte à pénétrer dans un tissu d'une paroi d'une cavité du coeur, cet accessoire d'implantation comprenant :
- un cathéter téléorientable (40) avec une lumière interne, prolongé à son extrémité distale par un embout (50) comprenant une embase (52) à laquelle se raccorde le cathéter (40) et une partie cylindrique (54) définissant un volume intérieur (56) apte à loger la capsule ; et
- un sous-cathéter (30) logé à l'intérieur de la lumière du cathéter téléorientable avec un degré de liberté en translation relative et un degré de liberté en rotation relative par rapport au cathéter téléorientable,
dans lequel le sous-cathéter et la capsule sont déployables téléscopiquement par rapport au cathéter entre i) une position rétractée où la capsule et son organe d'ancrage sont logés à l'intérieur de l'embout, et ii) une position déployée où la capsule est sortie de l'embout et est portée par l'extrémité distale du sous-cathéter, et l'extrémité distale du sous-cathéter et la région proximale (18) de la capsule sont pourvues de moyens découplables de solidarisation (20, 36) en translation et en flexion mutuelles, cet accessoire d'implantation étant **caractérisé en ce que** l'embout présente, entre son embase (52) et sa partie cylindrique (54), une partie souple (58 ; 158 ; 258) formant une articulation assurant entre l'embase et la partie cylindrique une déformabilité élastique en flexion et en compression.

2. L'accessoire d'implantation de la revendication 1, dans lequel ladite partie souple (58) présente la forme d'un manchon en matériau élastiquement déformable.

3. L'accessoire d'implantation de la revendication 2, dans lequel le manchon (58) est traversé par une série d'orifices (58a).

4. L'accessoire d'implantation de la revendication 2, dans lequel le manchon (258) comprend une zone en forme de soufflet.

5. L'accessoire d'implantation de la revendication 2, dans lequel ladite partie souple (158) présente la forme d'une cage.

6. L'accessoire d'implantation de la revendication 5, dans lequel la cage comprend deux bagues espacées (158a, 158b) de fixation à l'embase et à la partie cylindrique, et un ensemble de barres (158c) élastiquement déformables reliant les deux bagues l'une à l'autre dans une direction généralement axiale.

7. L'accessoire d'implantation de la revendication 1, dans lequel les contours extérieurs de la partie souple (58 ; 158 ; 258) s'étendent généralement dans le prolongement des contours de la partie cylindrique (54) dans la direction axiale de l'embout (50).

8. L'accessoire d'implantation de la revendication 1, dans lequel le sous-cathéter (30) présente, au droit de la partie souple (58) de l'embout, une région à déformation privilégiée (38) apte à faciliter la déformation élastique de ladite partie souple tout en préservant les efforts nécessaires à la manipulation de la capsule lors de rotations ou de translations.

9. L'accessoire d'implantation de la revendication 8, dans lequel la région à déformation privilégiée comprend un élément tubulaire formé par l'enroulement d'une spire, aux extrémités duquel s'arriment la partie principale (34) du sous-cathéter (30) et une partie (36) des moyens découplables de solidarisation avec la capsule (10).

10. L'accessoire d'implantation de la revendication 1, dans lequel l'embout de protection comprend de part et d'autre de la partie souple des marqueurs radio-opaques (59).

11. L'accessoire d'implantation de la revendication 1, dans lequel la partie souple est au moins partiellement radio-opaque.

## Patentansprüche

1. *In situ-*Implantations*z*ubehörteil für eine autonome intrakardiale Kapsel (10) umfassend einen zylindrischen rohrförmigen Körper (12), der an seinem distalen Ende mit einem Verankerungselement (14) versehen ist, das dazu geeignet ist, in ein Gewebe einer Wand einer Herzkammer einzudringen,
wobei dieses Implantationszubehörteil:
- einen fernausrichtbaren Katheter (40) mit einem internen Lumen umfasst, der an seinem distalen Ende durch ein Endstück (50) verlängert wird, welches einen Sockel (52) umfasst, an den sich der Katheter (40) und ein zylindrischer Teil (54) anschließen, wobei der zylindrische Teil einen Innenraum (56) umschließt, der dazu geeignet ist, die Kapsel aufzunehmen.
- einen Nebenkatheter (30), der im Inneren des Lumens des fernausrichtbaren Katheters mit einem Freiheitsgrad in Bezug auf die relative Translation und mit einem Freiheitsgrad in Bezug auf die relative Rotation zum fernausrichtbaren Katheter gelagert ist,
wobei der Nebenkatheter und die Kapsel relativ zum Katheter zwischen i) einer eingezogenen Position, in der die Kapsel und deren Verankerungselement im Inneren des Endstücks gelagert sind, und ii) einer ausgefahrenen Position, in der die Kapsel aus dem Endstück herausragt und vom distalen Ende des Nebenkatheters gehalten wird, teleskopisch ausgefahren werden können, und das distale Ende des Nebenkatheters und der proximale Bereich (18) der Kapsel mit zueinander verschiebbaren und biegbaren entkuppelbaren Befestigungsmitteln (20, 36) versehen sind,
wobei dieses Implantationszubehörteil **dadurch gekennzeichnet ist, dass** das Endstück zwischen seinem Sockel (52) und seinem zylindrischen Teil (54) einen flexiblen Teil (58; 158; 258) aufweist, der ein Gelenk bildet, das zwischen dem Sockel und dem zylindrischen Teil eine elastische Verformbarkeit durch Biegung und unter Druck gewährleistet.

2. Implantationszubehörteil nach Anspruch 1, bei dem der flexible Teil (58) nach Art einer Hülse aus einem elastisch verformbaren Material ausgebildet ist.

3. Implantationszubehörteil nach Anspruch 2, bei dem die Hülse (58) durch eine Reihe von Öffnungen (58a) durchquert ist.

4. Implantationszubehörteil nach Anspruch 2, bei dem die Hülse (258) einen balgförmigen Bereich aufweist.

5. Implantationszubehörteil nach Anspruch 2, bei dem der flexible Teil (158) in Form eines Käfigs ausgebildet ist.

6. Implantationszubehörteil nach Anspruch 5, bei dem der Käfig zwei voneinander beabstandete Ringe (158, 158b) zur Befestigung am Sockel und am zylindrischen Teil umfasst, sowie einen Satz von elastisch verformbaren Stäben (158c), die beide Ringe in einer allgemein axialen Richtung miteinander verbinden.

7. Implantationszubehörteil nach Anspruch 1, bei dem die Außenkonturen des flexiblen Teils (58, 158; 258) sich allgemein im Anschluss an die Konturen des zylindrischen Teils (54) in der axialen Richtung des Endstücks (50) erstrecken.

8. Implantationszubehörteil nach Anspruch 1, bei dem der Nebenkatheter (30) an dem flexiblen Teil (58) des Endstücks einen bevorzugten Verformungsbereich (38) aufweist, der dazu geeignet ist, die elastische Verformung des flexiblen Teils unter Bewahrung des zur rotatorischen oder translatorischen Handhabung der Kapsel notwendigen Aufwands zu erleichtern.

9. Implantationszubehörteil nach Anspruch 8, bei dem der bevorzugte Verformungsbereich ein durch eine aufgerollte Windung gebildetes rohrförmiges Element umfasst, an dessen Enden sich der Hauptteil (34) des Nebenkatheters (30) und ein Teil (36) der entkuppelbaren Mittel zur Befestigung an der Kapsel (10) ankoppeln.

10. Implantationszubehörteil nach Anspruch 1, bei dem das Schutzendstück auf beiden Seiten des flexiblen Teils radio-opake Marker (59) aufweist.

11. Implantationszubehörteil nach Anspruch 1, bei dem der flexible Teil zumindest teilweise radio-opak ist.

## Claims

1. An *in situ* implantation accessory of an autonomous intracardiac capsule (10) comprising a cylindrical tubular body (12) provided at its distal end with an anchoring member (14) adapted to penetrate into a tissue of a wall of a cavity of the heart,
the implantation accessory comprising:
- a remotely steerable catheter (40) with an inner lumen, extended at its distal end by a tip (50), the tip comprising a base (52) to which the catheter (40) connects to and a cylindrical portion (54) defining an interior volume (56) suitable for housing the capsule; and
- a sub-catheter (30) housed within the lumen of the remotely steerable catheter, the sub-catheter being moveable in translation and rotation relative to the remotely steerable catheter,
wherein the sub-catheter and the capsule are telescopically deployable with respect to the catheter between i) a retracted position wherein the capsule and the anchoring member are housed inside the tip, and ii) an extended position wherein the capsule is outside of the tip and is carried by a distal end of the sub-catheter, and the distal end of the sub-catheter and a proximal region (18) of the capsule are provided with disconnectable means of attachment (20, 36) in mutual translation and bending,
the implantation accessory being **characterized in that** the tip has, between the base (52) and the cylindrical portion (54), a flexible portion (58; 158; 258) forming an articulation between the base and the cylindrical portion providing an elastic deformability in bending and in compression.

2. The implantation accessory according to claim 1, wherein said flexible portion (58) has the shape of a sleeve made of an elastically deformable material.

3. The implantation accessory according to claim 2, wherein the sleeve (58) is traversed by a plurality of orifices (58a).

4. The implantation accessory according to claim 2, wherein the sleeve (258) comprises a bellows-shaped region.

5. The implantation accessory according to claim 2, wherein said flexible portion (158) has the shape of a cage.

6. The implantation accessory according to claim 5, wherein the cage comprises two spaced rings (158a, 158b) for fixing to the base and to the cylindrical portion, and a plurality of elastically deformable bars (158c) connecting the two rings to each other in a generally axial direction.

7. The implantation accessory according to claim 1, wherein the outer contours of the flexible portion (58; 158; 258) extend generally in continuation of the contours of the cylindrical portion (54) in the axial direction of the tip (50).

8. The implantation accessory according to claim 1, wherein the sub-catheter (30) has, in line with the flexible portion (58) of the tip, a preferred deformation region (38) adapted to facilitate the elastic deformation of the flexible portion while maintaining the effort required for manipulating the capsule by rotation and translation.

9. The implantation accessory according to claim 8, wherein the preferred deformation region comprises a tubular member formed by windings of a coil, wherein ends of the tubular member are coupled to a main portion (34) of said sub-catheter (30) and a portion (36) of the disconnectable means of attachment to the capsule (10).

10. The implantation accessory according to claim 1, wherein the protective tip comprises radiopaque markers (59) on either side of the flexible portion.

11. The implantation accessory of claim 1, wherein the flexible portion is at least partially radiopaque.
